# EUROPEAN PATENT SPECIFICATION

(11) **EP 3 197 297 B1**
(45) Date of publication and mention of the grant of the patent: **01.07.2020**
(21) Application number: 15781742.0
(22) Date of filing: 21.09.2015
(51) Int. Cl.: A23L 33/15, A23L 33/16, A23L 33/21, A61K 31/714, A61K 31/733, A61K 33/04, A61K 8/00, C09B 1/00

(54) **PREBIOTIC INULIN BASED PREPARATION**
PREPARAT AUF BASIS VON PREBIOTISCHES INULIN
PREPARATION A BASE D'INULINE PREBIOTIQUE

(30) Priority: 23.09.2014 IT MI20141648
(43) Date of publication of application: 02.08.2017
(73) Proprietor: Dom Terry International S.r.l., 20121 Milano (IT)
(72) Inventor: TERENZIO, Domenico, I-50144 Firenze (IT); RASTRELLI, Luca, I-50144 Firenze (IT); PARISELLA, Chiara, I-50144 Firenze (IT)
(74) Representative: Croce, Valeria
(86) International application number: PCT/IB2015/057265
(87) International publication number: WO 2016/046727

(56) References cited:
- WO-A1-02/39834
- WO-A1-2006/108208
- WO-A1-2015/105437
- WO-A2-02/47493
- CN-A- 103 330 118
- DE-A1- 10 206 995
- DATABASE MEDLINE [Online] US NATIONAL LIBRARY OF MEDICINE (NLM), BETHESDA, MD, US; 2011, MINUSHKIN O N ET AL: "[Fructooligo- and fructopoly-saccharides in correction and prevention of intestinal microbiocenosis disorders in patients with bronchopulmonary pathology receiving antibacterial therapy].", XP002739488, Database accession no. NLM21695955 & MINUSHKIN O N ET AL: "[Fructooligo- and fructopoly-saccharides in correction and prevention of intestinal microbiocenosis disorders in patients with bronchopulmonary pathology receiving antibacterial therapy].", EKSPERIMENTAL'NAIA I KLINICHESKAIA GASTROENTEROLOGIIA = EXPERIMENTAL & CLINICAL GASTROENTEROLOGY 2011, no. 3, 2011, pages 79-87, ISSN: 1682-8658
- DATABASE CAPLUS [Online] CHEMICAL ABSTRACTS SERVICE, COLUMBUS, OHIO, US; 21 July 2010 (2010-07-21), Malkov et al.: "Agent for the prevention of metabolic derangement", XP002739489, retrieved from STN Database accession no. 2010:900271 & RU 2 394 583 C1 (MALKOV MARK ABOVICH [RU]; DAN KOVA TAT JANA VASIL EVNA [RU]) 20 July 2010 (2010-07-20)
- DATABASE CAPLUS [Online] CHEMICAL ABSTRACTS SERVICE, COLUMBUS, OHIO, US; 2 October 2013 (2013-10-02), Gou: "Health care food fro promoting weight reduction", XP002739490, retrieved from stn Database accession no. 2013:1563359 & CN 103 330 118 A (GOU CHUNHU) 2 October 2013 (2013-10-02)
- DATABASE CAPLUS [Online] CHEMICAL ABSTRACTS SERVICE, COLUMBUS, OHIO, US; 23 January 2007 (2007-01-23), XP002751106, Database accession no. 2007:75196 & CN 1 895 067 A (GOU CHUNHU [CN]) 17 January 2007 (2007-01-17)

## Description

### STATE OF THE ART

Functional foods can be defined as natural or processed foods that, in addition to meeting the normal organoleptic and nutritional expectations, bring clear benefits to human health, preventing dysfunctions due to particular active ingredients from the physiological point of view.

The consumption of such foods, associated with a healthy lifestyle, can thus help to improve health and well-being, prevent some metabolic disorders and reduce the risk of disease.

In this context, flours enriched with prebiotic fibers and other molecules of a health-related interest find significant use.

The food industry produces a large amount of plant-derived by-products during the transformation processes; similarly, during the collection step, a selection of parts of the plants not requested by the food market and considered of little value is often performed.

Regarding the artichoke, the edible portion is very little by weight compared to the total biomass; in fact, it is only about 15-20% (by weight) of the plant and is especially represented by the "fleshy" portion of the flower head.

The amount of waste such as: external and intermediate bracts, stems and leaves, is therefore very large.

The presence of compounds like polyphenols, inulin and crude proteins is appreciable and also makes artichoke waste rather interesting for different sectors such as the pharmaceutical, cosmetic, nutraceutical and herbal industry, as well as for the production of animal feed, fertilizers and natural organic dyes.

### Inulin

Inulin is a polymer formed by fructose units and is the characteristic reserve substance of Asteraceae. The degree of polymerization determines the various types and different activity thereof.

It belongs to fructans or fructooligosaccharides (FOS), and is defined as a prebiotic fiber: in fact, it is not digested by humans as they do not possess the necessary enzymatic pattern.

In fact, studies prove that this has a selective nutritional activity towards the predominant intestinal microflora, especially bifidobacteria and lactobacilli, causing an increase in their number and activity, as well as the production of short-chain fatty acids that promote an increase in the fecal mass and proper bowel function; this effect also causes the elimination, by competitive mechanism, of the undesired flora in the intestinal tract.

Inulin also has an activity of prevention of colon cancer, it regulates the absorption of minerals such as calcium and blood levels of lipids, of which they facilitate the metabolism (Van Loo et al., 1999). Some studies show that in humans, induction in the production of butyrates by inulin causes an inhibition of cell growth, a controlled differentiation and a reduced metastasis of cancer agents.

In addition, it seems that inulin reduces exposure to carcinogenic and mutagenic agents and suppresses residual cancer cells.

It has been proven that since it does not allow the absorption of sugars, it can be used in foods for diabetics (Van Loo et al., 1999; Hellwege et al., 2000) .

The carbohydrates normally taken with the diet, which are absorbed as hexose sugars (glucose, fructose), have a calorie value of 3.9 kcal/g (16.3 kj/g) from which the cell metabolism produces 38 mol ATP/mol.

To the contrary, inulin and other fructose oligopolymers are not digested, i.e. not absorbed, and thus they do not provide any calorie value *(non-digestible oligosaccharides* - NDO) .

The edible portion of artichoke is characterized, despite the poor amount of sugars, by a large amount of inulin (75% of the total carbohydrate content) and other water-soluble polysaccharides.

In bracts, a by-product of the artichoke, the weight of inulin has been found to be greater than that found in chicory *(Cichorium intybus* L.) and it is approximately 30% of the total content.

Therefore, as regards the presence of inulin, the by-products of the artichoke can be considered a real resource.

### Vitamin B12

Cyanocobalamin (vitamin B12) is involved in homocysteine metabolism, which can reach too high values due to a reduced intake through diet, as it is present only in foods of animal origin and is therefore lacking in bakery products from cereals.

In addition, genetic predispositions that affect the synthesis of enzymes involved in its metabolism can result in a decreased absorption thereof. Homocysteine is a sulfur amino acid that derives from the transformation of methionine (essential amino acid) largely present in the diet. In the body, it circulates in the blood bound to albumin.

The presence of large homocysteine values is called hyperhomocysteinemia and is considered an important and independent risk factor for cardiovascular accidents.

The relationship between hyperhomocysteinemia and cardiovascular diseases was emphasized by numerous studies including the famous Framingham Heart Study.

### Selenium

Selenium is an element that acts mainly as a component of the antioxidant enzyme glutathione peroxidase.

Due to its ability to protect cell membranes from oxidation, selenium has a protective effect against cardiovascular diseases.

Low levels of selenium are linked to an increased risk of cancer, cardiovascular diseases, inflammatory diseases and other pathologies associated with the damage caused by free radicals, including premature aging and the formation of cataracts.

### Resveratrol

It belongs to the family of polyphenolic compounds, is a stilbene and is found in the berries of grape, in wine, in some berries and oil seeds (peanut) and in certain plants such as *Polygonum cuspidatum,* used in traditional Asian medicine to treat heart and liver conditions.

There are numerous pharmacological and clinical studies reported for this natural substance, which have stressed its antioxidant power against several species of free radicals and against oxidation of low density lipoproteins (LDL) and the subsequent inflammation phenomena at a vascular level.

It has an antiproliferative, immunostimulant and anti-inflammatory action.

### Hydroxytyrosol

A natural antioxidant, it is found in high concentrations in the vegetation water of olives and in lower concentrations also in olive leaves, it has immunostimulating and antibiotic properties.

### Coenzyme Q10

It is an endogenous lipophilic antioxidant involved in energy processes, due to its ability to transfer electrons and act as an antioxidant, it protects cells from free radical damage.

WO02/39834 discloses compositions comprising inulin, Vitamin B12 and selenium as macro-component to enrich various types of food products.

### BRIEF DESCRIPTION OF THE FIGURES

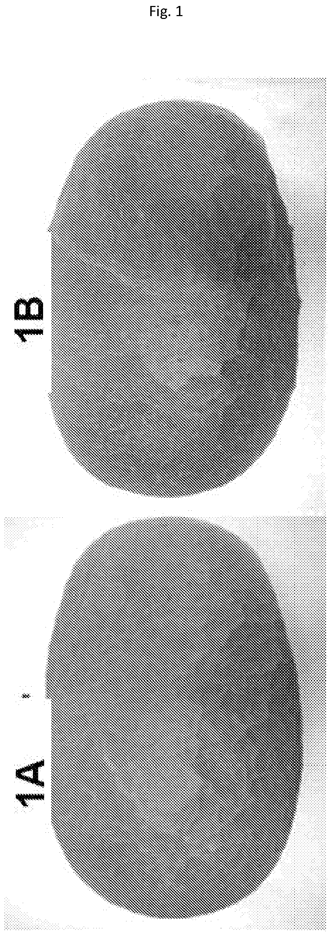

Figure 1A shows a product obtained with a traditional composition; Figure 1B shows an equivalent product obtained using the preparation of the invention.

### OBJECT OF THE INVENTION

A first object of the present invention, therefore, is a functional preparation according to claim 1 and dependent claim.

A process for the preparation of the composition of the invention is also disclosed.

In a second object, the invention describes flours, foods, feeds, fertilizers and dyes comprising the functional preparation of the invention.

In a further object, the use of the preparation in the pharmaceutical, nutraceutical and herbal industry is described.

In yet a further aspect, the invention describes the preparation of the present invention as a medicament and, in particular, to reduce the level of cholesterol, glucose, lipids, as an antioxidant and thus in the treatment of heart dysfunctions, hypertension, prevention of diseases associated with metabolic dysfunction, in the treatment of diabetes and in the prevention of cancer.

### DETAILED DESCRIPTION OF THE INVENTION

According to a first object of the invention, a functional preparation as per claim 1 is described. In particular, the three components mentioned above are in the following concentrations (weight/weight):

| | |
|---|---|
| inulin | 99.9975-99.998245% |
| vitamin B12 | 0.001685-0.002445% |
| selenium* | 0.000055-0.00007% |

Selenium is preferably added in the form of selenomethionine.

The composition indicated is to be understood as referred to a total equivalent to 100%; therefore, the greater the percentage of a component, the smaller the corresponding composition of another component.

According to a preferred aspect of the invention, the preparation of the invention has the following composition:

| **Ingredient** | **Composition on 100 g of powder** |
|---|---|
| INULIN | 99.9981 g |
| Vitamin B12 | 0.001833 g (1.83 mg) |
| Selenium | 0.000066 g (66.66 µg) |

In one aspect of the invention, the inulin used to prepare the composition is obtained from the artichoke plant and preferably, from parts that are discarded after the harvest or during the processing and transformation process as of little or no interest to the food industry, such as external bracts, intermediate bracts, stems and leaves.

Alternatively, other plants can be useful sources of inulin to use, such as garlic, asparagus root, white Salsify (*Tragopogon porrifolius*), dahlia tubers, chicory.

In particular, the inulin content in these sources can be as shown hereafter:

| Source | % inulin (weight/weight) |
|---|---|
| Garlic | 15-20 |
| Asparagus root | 10-15 |
| *Tragopogon porrifolius* | 15-20 |
| Dahlia tuber | 15-20 |
| Chicory | 15-20 |
| Jerusalem artichoke | 15-20 |

As regards the degree of polymerization of inulin, for the purposes of the present invention reference is made to a degree of polymerization of about 20.

An example of a commercially available product that has these features is the product Fibruline® instant. In one aspect of the invention, the composition described also comprises other functional substances that are able to interact with inulin, in particular with the hydroxyl groups, forming useful complexes.

Such substances are in particular selected from the group comprising: resveratrol, hydroxytyrosol, astaxanthin, coenzyme Q, germanium, preferably in organic form (i.e. complexed with suitable compounds), for example in the form of sesquioxide, inositol, flavonoids and other antioxidant molecules or with a functional activity.

The preparation of the invention may further comprise additional soluble or insoluble fibers.

Such components may be present in the following percentages by weight (with respect to 2 g of the composition object of the invention):

| **Compound** | **Percentage** |
|---|---|
| Resveratrol | 0.1% |
| Hydroxytyrosol | 0.3 % |
| Astaxanthin | 0.1% |
| Coenzyme Q10 | 0.1% |
| Germanium (sesquioxide) | 0.2% |
| Inositol | 0.3% |

| | |
|---|---|
| Flavonoids | 0.5% |

In one aspect of the invention, these substances are present in amounts of about 0.1-5% (weight/weight) and can be added in the form of dried titrated extracts.

A process for the preparation of the functional composition of the invention is also disclosed.

The preparation of the composition of the invention comprises an initial hydration step.

In particular, the water used is water treated by suitable methods which alter the pH thereof.

In a preferred aspect, the water used has a pH of about 8.5-9.5.

In an even more preferred aspect, the water has been treated by suitable methods which vary the molecular organization, so as to form hexagonal clusters.

In this step, the following components are added to water: vitamin B12 and selenium (in a suitable form, such as selenomethionine).

If envisioned by one of the further aspects of the invention, other components such as coenzyme Q10, astaxanthin, hydroxytyrosol, germanium (in the form of organic complex), inositol, flavonoids or other antioxidant molecules may also be added in this step. Preferably, the ingredients are mixed in the dark. Inulin is then added in a second step, preferably in the amount of about 80 g per liter of water, followed by suitable mixing.

The homogeneous preparation thus obtained is subjected to freeze-drying and then to drying to reduce the relative humidity to about 3%.

The process of the invention allows the vitamin B12 and selenium molecules, and possibly of other components, to be trapped within the mesh structure of inulin.

Freeze-drying is able not to alter such a structure, which is promptly restored by hydration prior to the use of the composition of the invention.

The preparation of the invention is described herein also for the production of many products:
- in the food industry:
   - beverages (milk and milk-based beverages, powdered milk preparations, coffee, tea, herbal teas, chocolate, soluble preparations for beverages, wine, fruit juices, tomato juice, etc.)
   - bakery products (both savory, such as bread, pizza, flat bread, breadsticks, crackers and also sweet such as: croissants and snacks in general, cakes or cookies);

   - meat products (hamburgers, meatballs, cured meats such as mortadella, salami, frankfurters, sausage, luncheon meat;
   - sauces and condiments (mayonnaise, ketchup, jelly, etc.);
   - preparations based on vegetal and animal fats based on butter, oil, lard, mascarpone cheese, cream and milk;
   - dairy products (milk, mozzarella, ricotta, cheese);
   - sweets and confectionery (candies, chewing gums, etc.), chocolate and chocolate preparations, ice cream, sorbets, frozen desserts and sauces, marmalades and jams, jellies;
   - confectionery (candies, chewing gums);
   - sweeteners;
   - special foods (for people suffering from celiac disease, for babies and children, for sportsmen, such as beverages and foods for replenishing sugars and mineral salts);
- in the livestock industry:
   - feeds and special feeds;
   - supplements;
- in the cosmetic industry:
   - creams, tonics, cleansers for the body, hair, shampoos
- in the agricultural industry:
   - fertilizers;
- in the industry:
   - dyes.

In one aspect of the invention, the preparation described is used for obtaining flours or flour mixtures, processed flours or preparations for use in making the above products.

The use of the mixture of the invention, in particular, can improve the technological and organoleptic properties thereof or impart particular functional properties to the food in which it is included.

To this end, the preparation may be preferably added to a flour in a percentage of about 2-8% (weight/weight) and preferably of about 4.5-5% (weight/weight) in order to impart technological improvements or to replace fats and/or emulsifiers.

Percentages of about 8-25% (weight/weight), however, can be used in order to impart marked preventive and/or therapeutic prebiotic actions to the final product.

Bakery preparations as described above can be made with the flours described.

Obviously, the use of flours and gluten-free flours, therefore suitable for people suffering from celiac disease, such as Quinoa, buckwheat, kamut, rice flour, for example, is equally contemplated for the purposes of the present invention.

The use of vegetable-based proteins or other vegetable fibers, on the other hand, allows the production of foods for vegetarians.

In a preferred aspect of the invention, the following amounts of preparation of the invention are used to obtain the following products with respect to the quantity of flour used:

| **Product** | **% weight** |
|---|---|
| Pizza | 5-8 |
| Bread | 5-8 |
| Pasta | 6-7 |

The same use can be extended for confectionery products as described above.

Moreover, meat-based food preparations and beverages can also be obtained.

In particular, a beverage can be obtained using an amount of the composition of the invention of about 1.5% (weight/volume of water of the beverage).

As regards, instead, the meat-based preparation, these preparations will comprise about 6.5% of the preparation of the invention (weight/weight of the meat).

As indicated above, in the food preparations described, the use of the composition of the invention can replace at least partially the use of fats and/or emulsifiers.

For the present purposes, "fats" refers to the total content by weight of vegetable and animal fats used and, therefore, includes butter, oils, lard, mascarpone cheese, cream and milk.

In a further aspect, the present patent application discloses the use of the preparation of the invention to increase the bioavailability of vitamin B12 and selenium.

The addition of selenium to the flour through an inulin-enriched mixture surprisingly favors its organication by bacteria, which transform part of the admixed selenium (SeIV) into more bioavailable organic forms such as SeMet and MeSeCys and partly into other organic fragments.

In the light of the above, the preparation of the invention finds use in the livestock industry for the preparation of feeds, in the dye industry, in the pharmaceutical industry, in the nutraceutical industry, in the cosmetics industry, in the food supplements industry and in the herbal industry, in the agricultural field.

In the latter application, the preparation of the invention has been shown to promote the translocation at a stomatal level and the stability on the leaf cuticle.

Feeds, dyes, pharmaceuticals, nutraceuticals, supplements, herbal preparations and all the products comprising the composition described above are all further objects of the invention.

According to a further aspect, the claimed preparation finds use as a medicament.

In particular, its ability to reduce the level of cholesterol, glucose, lipids, oxidizing agents has been demonstrated (thereby carrying out an antioxidant function) and, therefore, its use in the treatment of cardiac dysfunctions, hypertension, diabetes and diseases associated with metabolic dysfunction is contemplated herein.

By the term "metabolic syndrome", in particular, it is meant the set of metabolic risk factors that increase the possibility of developing heart diseases, stroke and diabetes.

The exact cause of the metabolic syndrome is not known, although there is evidence of genetic factors, the presence of an excessive amount of body fat and lack of exercise.

The use of the preparation of the invention for the prevention of tumors is also described herein.

### EXAMPLE 1

### INUPLUS composition

| **Ingredient** | **Composition on 100 g of powder** |
|---|---|
| INULIN | 99.9981 g |
| Vitamin B12 | 0.001833 g (1.83 mg) |
| Selenium | 0.000066 g (66.66 µg) |

### EXAMPLE 2

The composition of the invention was used for the preparation of a flour and, therefore, of bakery products such as bread and pizza.

Figure 1 shows a loaf of bread prepared with a traditional composition, not containing the preparation of the invention (1A) and a loaf of bread obtained using the preparation of the invention (1B).

Figure 1B shows an improvement of the color of the bakery product after baking which is accompanied by a greater crispness thereof.

### EXAMPLE 3

The composition of the invention was used for the preparation of milk-based products: milk, ice cream, yoghurt, mozzarella, ricotta, cheese, in an amount of about 6-7% by weight with respect to the volume of milk.

### EXAMPLE 4

The composition of the invention was used for the preparation of beverages.

| **Product** | **% weight (weight/volume of water)** |
|---|---|
| wine | 3.5% |
| water | 1.5% |

### EXAMPLE 5

The composition of the invention was used for the preparation of some foods.

| **Product** | **% weight (weight/weight of chocolate)** |
|---|---|
| chocolate | 8% |

| **Product** | **% (weight/weight of meat)** |
|---|---|
| hamburgers, meatballs, cured meats, frankfurters, sausage, luncheon meat | 5-7% |

The advantages offered by the preparation of the present invention are clear from the above.

First, it allows using and valuing a resource, represented by the waste parts of the artichoke plant, which would otherwise be discarded.

The possibility of using other plant sources, such as chicory, therefore allows valuing other resources, which otherwise remain undervalued.

Furthermore, the combination of the three components: inulin, vitamin B12 and selenium, has been shown to produce a surprising synergistic effect with respect to the single ingredients.

This synergy was even more pronounced upon the addition of the other functionalizing substances described.

In particular, the possibility of using the composition of the invention as a texturing factor in the preparation of food products and, in particular, of sweet and savory bakery products has been shown. It has been noticed that during the process of fermentation and baking, the formation of highly stable organic and inorganic selenium complexes (such as SeMet and MeSeCys or other organic fragments) takes place, accompanied by an increase in the action of digestive enzymes.

The bioavailability of selenium and of other antioxidant and nutritional compounds is therefore increased and improved.

This is also possible due to the formation of a three-dimensional inulin lattice during the preparation of the mixture of the invention (facilitated by the use of water at a certain pH), in the meshes of which the other components are inserted, which can thus be more easily assimilated. Moreover, it has been seen that the preparation described above can replace, at least partly, the fat portion in bakery or dairy products, thus reducing the calories intake.

This is due to the possibility of forming water or milk microcrystals therebetween, which are not perceptible to the palate but allow obtaining a markedly creamy and smooth texture.

The neutral or slightly sweet taste (inulin has a sweetening power of about 30-50% compared to sucrose), moreover, allows its addiction without substantially altering the flavor of food.

The use of the preparation of the invention as a medicament, moreover, responds to the consumers' demand for natural products and nutraceuticals with hypolipidaemic, hypoglycemic and hypocholesterolemic action.

With particular reference to the levels of blood sugar, the surprising results obtained from the use of the preparation of the invention in obtaining products characterized by a lower glycemic index should be recognized.

Moreover, thanks to the consumption of the described preparation, it will be easier to get the recommended daily requirement of vitamin B12 (1-2 pg/day) and selenium (about 55 pg/day).

It was also surprisingly found that all the advantages above described are not in any way at the expense of other desirable properties in the final products obtained with the preparation of the invention, such as for example the possibility of providing a food product that is:
- more complete,
- more digestible, with particular reference to the digestibility and tolerance to lactose,
- more functional,
- crispier and more fragrant,
- looking more pleasing to the consumer,
- able to be preserved for as long as or even longer than the products which do not comprise the preparation of the invention, despite the ability of the compound to maintain a higher humidity,
- able to improve the capacity of absorption of calcium.

From the industrial point of view, the preparation of the invention improves the processability of the mixtures in which it is included, just for the fact of being able to increase the miscibility of the ingredients and, therefore, the formation of more homogeneous preparations.

In addition, with regard to bakery products, it was noted that the stability to baking processes has increased.

The composition of the invention has also surprisingly shown to improve the absorption of calcium in milk-based products, which is an important and useful element for health and well-being, especially for women, children and the elderly.

With particular reference to yoghurt, an additional synergistic action has been observed due to the combination with the yoghurt bacilli, which allows a more rapid colonization of the intestine and a greater percentage of survival of the strains introduced, at the same time improving the digestibility and tolerability of lactose, present although in a smaller amount compared to milk.

As regards the preparation of beverages, like for example water (understood as added and/or modified water beverages) or wine (understood as wine-based preparation), these are extremely useful in the diet of hospitalized patients that otherwise have difficulties in being fed due to problems in eating food.

On the contrary, the possibility of producing meat-based preparations, such as hamburgers, comprising the composition of the invention, meets the needs of being able to administer/take an adequate amount of fibers within a functional preparation which has synergies effects due to the association with vitamin B12 and selenium.

Those skilled in the art may make changes, additions or replacements of elements with others functionally equivalent from the description of the present invention in order to meet specific incidental needs, without departing from the scope of the appended claims.

Each of the features described as belonging to a possible embodiment can be obtained irrespective of the other embodiments described.

## Claims

1. Functional preparation comprising (percentage by weight on the total weight of the preparation):
| | |
|---|---|
| inulin | 99.9975-99.998245% |
| vitamin B12 | 0.001685-0.002445% |
| selenium | 0.000055-0.00007% |
wherein inulin has a degree of polymerization of 20.

2. Functional preparation according to claim 1, comprising:
| **Ingredients** | **weight on 100 g of powder** |
|---|---|
| inulin | 99.9981 g |
| vitamin B12 | 0.001833 g (1.83 mg) |
| selenium | 0.000066 g (66.66 µg) |

3. Functional preparation according to any one of claims 1 or 2, further comprising one or more functionalizing substances selected from the group comprising resveratrol, hydroxytyrosol, coenzyme Q, astaxanthin, germanium, possibly in organic form, inositol, flavonoids.

4. Functional preparation according to any one of claims 1 to 3, wherein inulin is obtained from artichoke or from other plant sources selected from the group comprising: garlic, asparagus root, white Salsify (Tragopogon porrifolius), dahlia tubers, chicory.

5. Functional preparation according to any one of claims 1 to 6, wherein said selenium is in the form of selenomethionine.

6. A flour comprising the functional preparation according to any of claims 1 to 5.

7. Flour according to the preceding claim, comprising from 2-8% and preferably 4.5-5% (weight /total weight of the flour) of the composition according to any of claims 1 to 5.

8. Food preparation obtained using the functional preparation according to any of claims 1 to 5, represented by a bakery product selected from the group comprising: savory products, such as bread, pizza, flat bread, breadsticks , crackers or sweet products, such as: croissants and snacks in general, cakes or cookies; or represented by sauces and condiments selected from the group comprising: mayonnaise, ketchup, jelly; or represented by cakes and confectionery products selected from the group comprising: candies, chewing gums; or represented by chocolate and chocolate preparations, ice cream, sorbets, frozen desserts, sauces, jams and marmalades, jellies; or represented by confectionery selected from the group which includes candies, chewing gums; or represented by sweeteners.

9. Food preparation represented by the pizza or bread according to the preceding claim, obtained using an amount of the functional preparation according to any of claims 1 to 5 of 5-8% (weight /weight of flour).

10. Food preparation obtained using the functional preparation according to any of claims 1 to 5, selected from the group comprising dairy products, milk, mozzarella, ricotta, cheese, yoghurt, ice cream, or preparations made from vegetable and animal fats made with butter, oil, lard, mascarpone cheese, cream and milk.

11. The food preparation according to the preceding claim, wherein said preparation selected from the group comprising dairy products, milk, mozzarella, ricotta, cheese, yoghurt and ice cream, comprises 6-7% (weight /volume of milk) of the preparation according to any of claims 1 to 5.

12. A meat-based food preparation obtained using the functional preparation according to any of claims 1 to 5 selected from the group comprising: hamburgers, meatballs, cured meats, frankfurters, sausage, luncheon meat.

13. A beverage obtained using the functional preparation according to any of claims 1 to 5, selected from the group comprising: milk and milk-based drinks, powdered milk preparations, coffee, tea, herbal teas, chocolate, soluble preparations for beverages, wine, fruit juices, tomato juice.

14. Use of the preparation according to any one of claims 1 to 5 in the pharmaceutical, nutraceutical, herbal industry, for the preparation of cosmetic products selected from the group comprising: creams, tonics, cleansers for the body, hair, shampoos, for the preparation of livestock products selected from the group comprising feeds and special feeds, supplements, for the preparation of fertilizers, for the preparation of dyes.

15. Preparation according to any one of claims 1 to 5, for use as a medicament.

16. Preparation according to any one of claims 1 to 5, for use as a medicament to reduce the level of cholesterol, glucose, lipids, as an antioxidant, in the prevention and treatment of cardiac dysfunctions, hypertension, diseases associated with metabolic dysfunction and diabetes and in the prevention of tumors.

## Patentansprüche

1. Funktionelle Zubereitung, umfassend (Gew.-% bezogen auf das Gesamtgewicht der Zubereitung):
| | |
|---|---|
| Inulin | 99,9975-99,998245 % |
| Vitamin B12 | 0,001685-0,002445 % |
| Selen | 0,000055-0,00007 % |
wobei Inulin einen Polymerisationsgrad von 20 aufweist.

2. Funktionelle Zubereitung nach Anspruch 1, umfassend:
| **Inhaltsstoffe** | **Gewicht bezogen auf 100 g Pulver** |
|---|---|
| Inulin | 99,9981 g |
| Vitamin B12 | 0,001833 g (1,83 mg) |
| Selen | 0,000066 g (66,66 µg) |

3. Funktionelle Zubereitung nach einem der Ansprüche 1 oder 2, ferner umfassend eine oder mehrere funktionalisierende Substanzen, ausgewählt aus der Gruppe, umfassend Resveratrol, Hydroxytyrosol, Coenzym Q, Astaxanthin, Germanium, möglicherweise in organischer Form, Inosit, Flavonoide.

4. Funktionelle Zubereitung nach einem der Ansprüche 1 bis 3, wobei Inulin erhalten ist aus Artischocke oder aus anderen pflanzlichen Quellen, ausgewählt aus der Gruppe, umfassend: Knoblauch, Spargelwurzel, Haferwurzel (Tragopogon porrifolius), Dahlienknollen, Chicoree.

5. Funktionelle Zubereitung nach einem der Ansprüche 1 bis 4, wobei das Selen in der Form von Selenmethionin vorliegt.

6. Mehl, umfassend die funktionelle Zubereitung nach einem der Ansprüche 1 bis 5

7. Mehl nach dem vorhergehenden Anspruch, umfassend 2-8 % und bevorzugt 4,5-5 % (Gewicht/Gesamtgewicht des Mehls) der Zusammensetzung nach einem der Ansprüche 1 bis 5.

8. Lebensmittelzubereitung, erhalten durch Verwenden der funktionellen Zubereitung nach einem der Ansprüche 1 bis 5, dargestellt durch ein Backprodukt, ausgewählt aus der Gruppe, umfassend: Schmackhafte Produkte, wie Brot, Pizza, Fladenbrot, Brotstangen, Cracker oder süße Produkte, wie: Corissants und Snacks im Allgemeinen, Kuchen oder Kekse; oder dargestellt durch Soßen und Gewürze, ausgewählt aus der Gruppe, umfassend: Mayonnaise, Ketchup, Gelee; oder dargestellt durch Kuchen und Süßwarenprodukte, ausgewählt aus der Gruppe umfassend: Süßigkeiten, Kaugummis; oder dargestellt durch Schokolade und Schokoladenzubereitungen, Eiscreme, Sorbets, gefrorene Desserts, Soßen, Konfitüren und Marmeladen, Gelees; oder dargestellt durch Süßwaren, ausgewählt aus der Gruppe, die Süßigkeiten, Kaugummis einschließt; oder dargestellt durch Süßungsmittel.

9. Lebensmittelzubereitung dargestellt durch die Pizza oder das Brot nach dem vorgehenden Anspruch, erhalten durch Verwenden einer Menge der funktionellen Zubereitung nach einem der Ansprüche 1 bis 5 von 5-8 % (Gewicht/Gewicht des Mehls).

10. Lebensmittelzubereitung, erhalten durch Verwenden der funktionellen Zubereitung nach einem der Ansprüche 1 bis 5, ausgewählt aus der Gruppe, umfassend Milchprodukte, Milch, Mozzarella, Ricotta, Käse, Joghurt, Eiscreme oder Zubereitungen hergestellt aus pflanzlichen und tierischen Fetten hergestellt mit Butter, Öl, Schmalz, Mascarpone, Käse, Sahne und Milch.

11. Lebensmittelzubereitung nach dem vorhergehenden Anspruch, wobei die Zubereitung, ausgewählt aus der Gruppe, umfassend Milchprodukte, Milch, Mozzarella, Ricotta, Käse, Joghurt und Eiscreme, 6-7 % (Gewicht/Volumen von Milch) der Zubereitung nach einem der Ansprüche 1 bis 5 umfasst.

12. Lebensmittelzubereitung auf Fleisch-Basis, erhalten durch Verwenden der funktionellen Zubereitung nach einem der Ansprüche 1 bis 5, ausgewählt aus der Gruppe, umfassend: Hamburger, Fleischbällchen, geräuchertes Fleisch, Frankfurter, Wurst, Frühstücksfleisch.

13. Getränk, erhalten durch Verwenden der funktionellen Zubereitung nach einem der Ansprüche 1 bis 5, ausgewählt aus der Gruppe, umfassend Milch und Getränke auf Milchbasis, Milchpulverzusammensetzungen, Kaffee, Tee, Kräutertees, Schokolade, lösliche Zubereitungen für Getränke, Wein, Fruchtsäfte, Tomatensaft.

14. Verwendung der Zubereitung nach einem der Ansprüche 1 bis 5 in der pharmazeutischen, nutrazeutischen, pflanzlichen Industrie, zur Herstellung von kosmetischen Produkten, ausgewählt aus der Gruppe, umfassend: Cremes, Tonika, Reinigungsmittel für den Körper, für die Haare, Shampoos, zur Herstellung von Viehprodukten, ausgewählt aus der Gruppe, umfassend Futtermittel und Spezialfuttermittel, Ergänzungsmittel, zur Herstellung von Düngemitteln, zur Herstellung von Farbstoffen.

15. Zubereitung nach einem der Ansprüche 1 bis 5 zur Verwendung als ein Medikament.

16. Zubereitung nach einem der Ansprüche 1 bis 5 zur Verwendung als ein Medikament zur Verringerung des Spiegels von Cholesterol, Glukose, Lipide, als ein Antioxidans, bei der Vorbeugung und Behandlung von kardialen Dysfunktionen, Hypertension, Krankheiten in Verbindung mit metabolischer Dysfunktion und Diabetes und bei der Vorbeugung von Tumoren.

## Revendications

1. Préparation fonctionnelle comprenant (pourcentage en poids/le poids total de la préparation) :
| | |
|---|---|
| inuline | 99,9975-99,998245% |
| vitamine B12 | 0,001685-0,002445% |
| sélénium | 0,000055-0,00007% |
dans laquelle l'inuline a un degré de polymérisation de 20.

2. Préparation fonctionnelle selon la revendication 1, comprenant :
| **Ingrédients** | **poids /100 g de poudre** |
|---|---|
| inuline | 99,9981 g |
| vitamine B12 | 0,001833 g (1,83 mg) |
| sélénium | 0,000066 g (66,66 µg) |

3. Préparation fonctionnelle selon l'une quelconque des revendications 1 et 2, comprenant en outre une ou plusieurs substances de fonctionnalisation choisies dans le groupe comprenant le resvératrol, l'hydroxytyrosol, la coenzyme Q, l'astaxanthine, le germanium, éventuellement sous forme organique, l'inositol, les flavonoïdes.

4. Préparation fonctionnelle selon l'une quelconque des revendications 1 à 3, dans laquelle l'inuline est obtenue à partir d'artichaut ou à partir d'autres sources végétales choisies dans le groupe comprenant : l'ail, les griffes d'asperge, le Salsifis blanc *(Tragopogon porrifolius),* les tubercules de dahlias, la chicorée.

5. Préparation fonctionnelle selon l'une quelconque des revendications 1 à 6, dans laquelle ledit sélénium est sous forme de sélénométhionine.

6. Farine comprenant la préparation fonctionnelle selon l'une quelconque des revendications 1 à 5.

7. Farine selon la revendication précédente, comprenant de 2-8% et, de préférence, de 4,5-5% (poids/poids total de la farine) de la composition selon l'une quelconque des revendications 1 à 5.

8. Préparation alimentaire obtenue en utilisant la préparation fonctionnelle selon l'une quelconque des revendications 1 à 5, représentée par un produit de boulangerie choisi dans le groupe comprenant : les produits salés, tels que : pain, pizza, pain plat, gressins, crackers, ou les produits sucrés, tels que : viennoiseries et collations en général, gâteaux ou biscuits ; ou représentée par les sauces et les condiments choisis dans le groupe comprenant : la mayonnaise, le ketchup, la gelée ; ou représentée par les gâteaux et les produits de confiserie choisis dans le groupe comprenant : les bonbons, les gommes à mâcher ; ou représentée par le chocolat et les préparations au chocolat, la crème glacée, les sorbets, les desserts glacés, les sauces, les confitures et marmelades, les gelées ; ou représentée par la confiserie choisie dans le groupe qui englobe les bonbons, les gommes à mâcher ; ou représentée par les édulcorants.

9. Préparation alimentaire représentée par la pizza ou le pain selon la revendication précédente, obtenue en utilisant une quantité de la préparation fonctionnelle selon l'une quelconque des revendications 1 à 5 de 5-8% (poids/poids de la farine).

10. Préparation alimentaire obtenue en utilisant la préparation fonctionnelle selon l'une quelconque des revendications 1 à 5, choisie dans le groupe comprenant les produits laitiers, le lait, la mozzarella, la ricotta, le fromage, le yaourt, la crème glacée, ou les préparations confectionnées à partir de graisses végétales et animales faites avec du beurre, de l'huile, du saindoux, du fromage mascarpone, de la crème et du lait.

11. Préparation alimentaire selon la revendication précédente, où ladite préparation, choisie dans le groupe comprenant les produits laitiers, le lait, la mozzarella, la ricotta, le fromage, le yaourt et la crème glacée, comprend 6-7% (poids/volume de lait) de la préparation selon l'une quelconque des revendications 1 à 5.

12. Préparation alimentaire à base de viande obtenue en utilisant la préparation fonctionnelle selon l'une quelconque des revendications 1 à 5, choisie dans le groupe comprenant : les hamburgers, les boulettes de viande, la charcuterie, les saucisses de Francfort, la saucisse, le pâté de viande.

13. Boisson obtenue en utilisant la préparation fonctionnelle selon l'une quelconque des revendications 1 à 5, choisie dans le groupe comprenant : le lait et les boissons lactées, les préparations au lait en poudre, le café, le thé, les tisanes, le chocolat, les préparations solubles pour boissons, le vin, les jus de fruit, le jus de tomate.

14. Utilisation de la préparation selon l'une quelconque des revendications 1 à 5 dans l'industrie pharmaceutique, nutraceutique, l'industrie des plantes médicinales, pour la préparation de produits cosmétiques choisis dans le groupe comprenant : les crèmes, les toniques, les produits nettoyants pour le corps, les cheveux, les shampoings, pour la préparation de produits pour bétail choisis dans le groupe comprenant les aliments pour animaux et les aliments spéciaux, les suppléments, pour la préparation d'engrais, pour la préparation de colorants.

15. Préparation selon l'une quelconque des revendications 1 à 5, destinée à être utilisée comme médicament.

16. Préparation selon l'une quelconque des revendications 1 à 5, destinée à être utilisée comme médicament pour réduire le taux de cholestérol, de glucose, de lipides, comme antioxydant, dans la prévention et le traitement des dysfonctionnements cardiaques, de l'hypertension, des maladies associées à un dysfonctionnement métabolique et au diabète et dans la prévention des tumeurs.
